# EUROPEAN PATENT SPECIFICATION

(11) **EP 2 004 163 B1**
(45) Date of publication and mention of the grant of the patent: **17.09.2014**
(21) Application number: 07781370.7
(22) Date of filing: 05.04.2007
(51) Int. Cl.: A61K 31/132, A61K 31/136, A61K 31/282, A61K 31/436, A61K 31/437, A61K 31/475, A61K 31/5375, A61K 31/573, A61K 31/675, A61K 31/704, A61K 31/7068, A61K 31/7076, A61K 45/06, A61P 35/00, A61P 35/02

(54) **COMBINATION OF EVEROLIMUS AND VINORELBINE**
KOMBINATION VON EVEROLIMUS UND VINORELBIN
COMBINAISON D'ÉVÉROLIMUS AVEC DE LA VINORELBINE

(30) Priority: 05.04.2006 US 789400 P
(43) Date of publication of application: 24.12.2008
(60) Divisional application: 13152306.0
(73) Proprietor: Novartis Pharma AG, 4056 Basel (CH); Novartis Pharma GmbH, 1230 Wien (AT)
(72) Inventor: BURKE, Gregory, Randolph, New Jersey 07869 (US); LANE, Heidi, CH-4105 Biel-Benken (CH); LINNARTZ, Ronald Richard, Andover, New Jersey 07821 (US); VERSACE, Richard William, Wanaque, New Jersey 07465 (US)
(74) Representative: Roth, Peter Richard
(86) International application number: PCT/US2007/066010
(87) International publication number: WO 2007/124252

(56) References cited:
- WO-A-02/098416
- WO-A-2006/039414
- WO-A-2006/065780
- WO-A-2007/056118
- WO-A-2007/057457
- WO-A-2007/106503
- RECHER, C; DOS SANTOS, C; DEMUR, C; PAYRASTRE, B: "mTOR, a new therapeutic target in acute myeloid leukemia" CELL CYCLE, vol. 4, no. 11, 2005, pages 1538-1547, XP002467483

## Description

The present disclosure relates to a combination comprising an mTOR inhibitor; and one or more pharmaceutically active agents; pharmaceutical compositions comprising said combination; methods of treatment comprising said combination; processes for making said combination; and a commercial package comprising said combination.

### Background of the Invention

Rapamycin and rapamycin derivatives are inhibitors of mTOR activity and exhibit a wide array of biological activities. It is also known that different combinations of active ingredients may increase anti-tumor behaviour. Therefore, there is a continuing need for new combinations of rapamycin and rapamycin derivatives, especially 40-O-(2-hydroxyethyl)rapamycin. WO02/098416A discloses the use of a mTOR inhibitor, in particular CCI-779, in combination with an anti-neoplastic alkylating agent in the treatment of neoplasms. WO2006/039414A discloses methods for treating AML which comprises administering a mTOR inhibitor to a patient in need thereof. WO2006/065780A discloses inter alia combinations of a comptothecin derivative and pharmaceutical agents selected from a microtubule active agent; an alkylating agent; an anti-neoplastic anti-metabolite, a platin compound; a topoisomerase II inhibitor, a VEGF inhibitor, a tyrsosine kinase inhibitor; an EGFR kinase inhibitor, an mTOR kinase inhibitor; an insulin-like growth factor I inhibitor; a Raf kinase inhibitor; a monoclonal antibody; a proteasome inhibitor; a HDAC inhibitor and ionizing radiation. Such combination therapies are disclosed to be useful for treating patients suffering from proliferative diseases or diseases associated with persistent angiogenesis. WO2007/056118A disclosed anti-neoplastic combinations with an mTOR inhibitor, such as in particular temsirolimus, and herceptin or HKI272. A combination of herceptin and HKI-272 is also provided. Recher et al, Cell Cycle 4:11, 2005, pages 1538-1547 disclose mTOR as new therapeutic target in AML and discloses drugs or small molecule inhibitors, which have been used in various in vitro and/or in vivo models in association with rapamycin or its analogues wherein synergistic or additive effect of mTOR inhibition was observed, or also neither synergism nor additive effect of mTOR inhibition. WO2007/057457A discloses methods for treating endocrine tumors by administration of an mTOR inhibitor, such as 40-O-(2hydroxyethyl)-rapamycin, optionally in combination with another drug. WO2007/106503A relates to a method for manufacturing a medicament intended for treating NSCL, pancreatic, colon or breast cancer tumors or tumor metastases in combination with an mTOR inhibitor.

### Summary of the Invention

The present invention provides a combination which comprises:
(a) everolimus; and
(b) vinorelbine.

In another aspect he present invention further provides a pharmaceutical compositions comprising:
(a) everolimus;
(b) vinorelbine; and
(c) a pharmaceutically acceptable carrier.

In another aspect the present invention further provides a commercial package or product comprising:
(a) everolimus; and
(b) a pharmaceutical formulation of vinorelbine for simultaneous, concurrent, separate or sequential use.

The combination partners (a) and (b) can be administered together, one after the other or separately in one combined unit dosage form or in two separate unit dosage forms. The unit dosage form may also be a fixed combination.
Combinations according to the present invention include fixed combinations, in which everolimus and vinorelbine are in the same formulation; kits, in which everolimus and vinorelbine in separate formulations are provided in the same package, e.g. with instruction for co-administration; and free combinations in which everolimus and vinorelbine are packaged separately, but instruction for concomitant or sequential administration are given.

In another aspect the present invention provides
- A pharmaceutical package comprising a everolimus and vinorelbine, beside instructions for combined administration;
- A pharmaceutical package comprising everolimus beside instructions for combined administration with vinorelbine;
- A pharmaceutical package comprising vinorelbine beside instructions for combined administration with everolimus;
which is a commercial package or product.

In another aspect the present invention provides a method of preventing or treating proliferative diseases or diseases that are associated with or triggered by persistent angiogenesis in a mammal, particularly a human, with a combination comprising:
(a) everolimus; and
(b) vinorelbine.

In another aspect the present disclosure provides the use of a combination, pharmaceutical composition or commercial package provided by the present invention for the manufacture of a medication for preventing or treating proliferative diseases or diseases that are associated with or triggered by persistent angiogenesis in a mammal.

### Brief Description of the Drawings

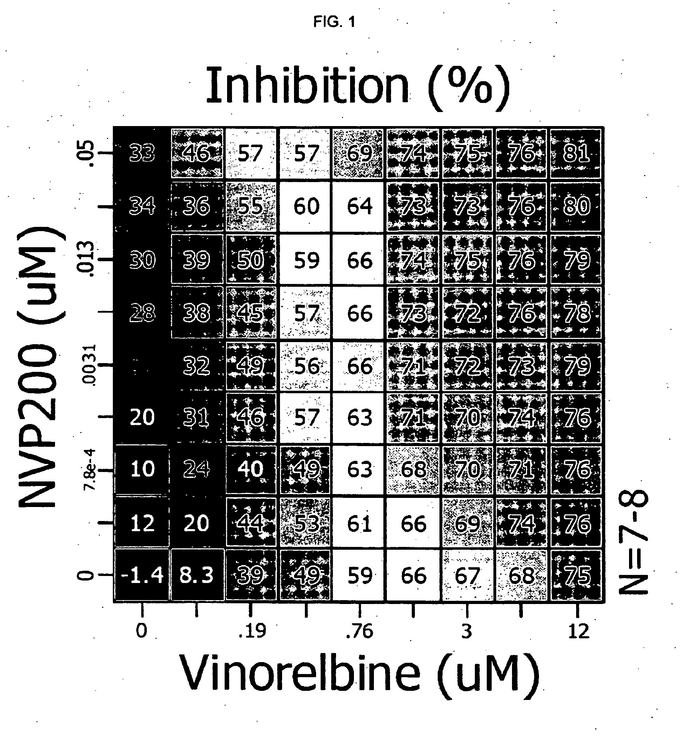

**FIG 1****:** shows the percent inhibition for a 81-point 9x9 dose matrix for the combination with 40-O-(2-hydroxyethyl)rapamycin and vinorelbine in A549 cells

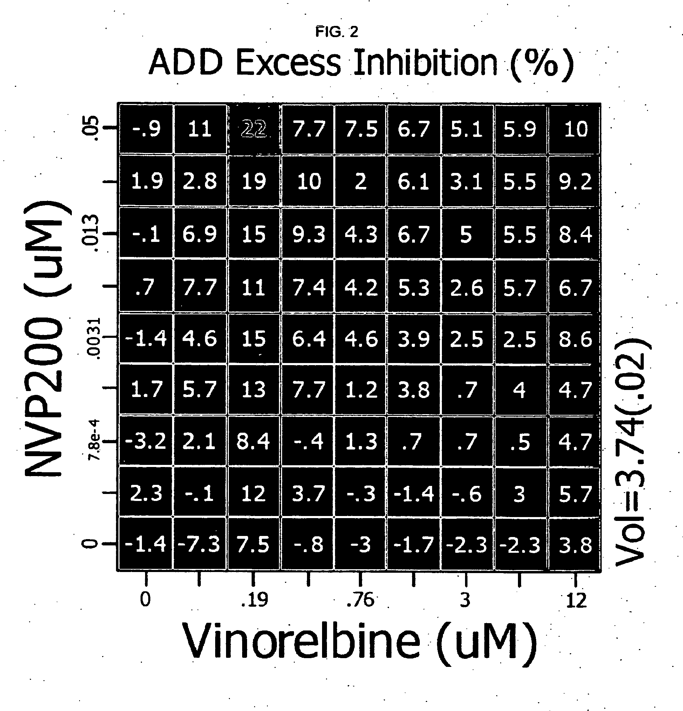

**FIG 2****:** shows the synergy for each dose point compared to the Loewe additivity model for the combination with 40-O-(2-hydroxyethyl)rapamycin and vinorelbine in A549 cells

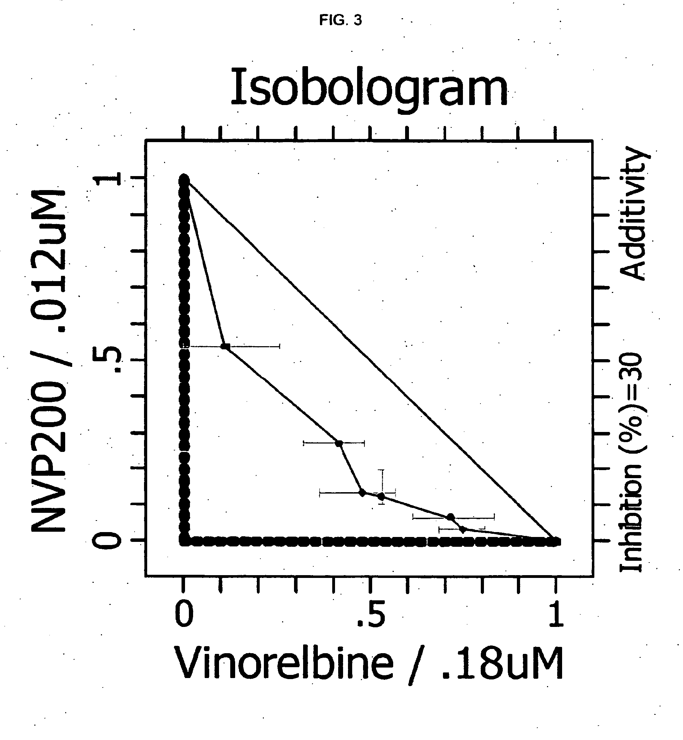

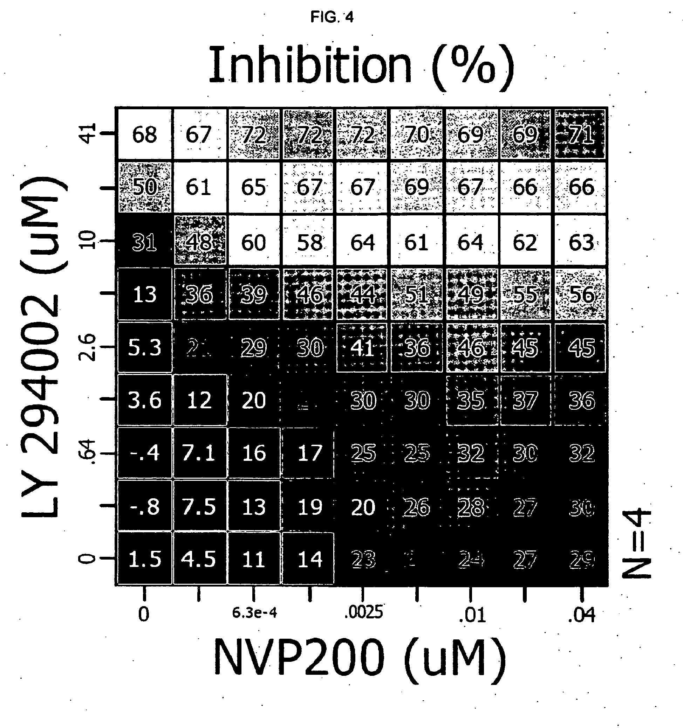

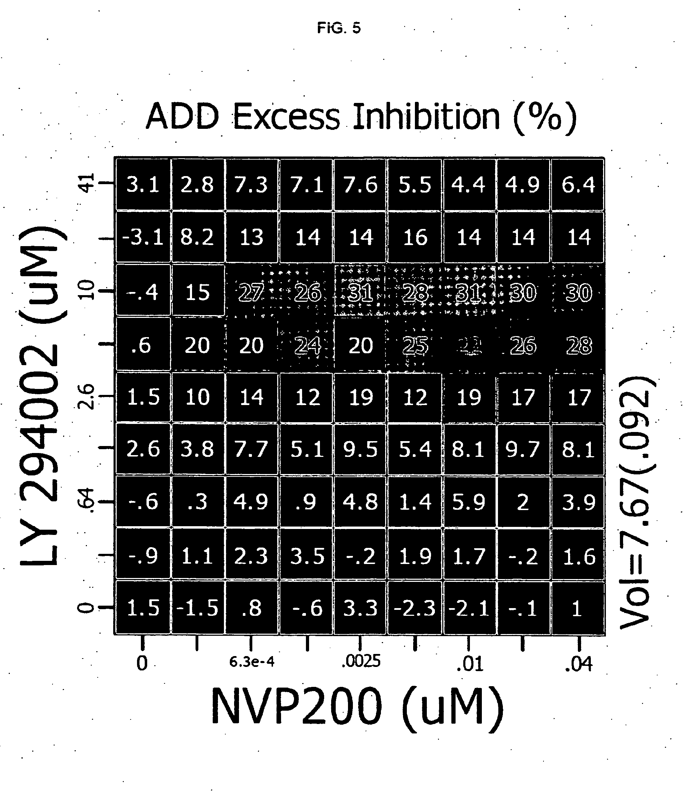

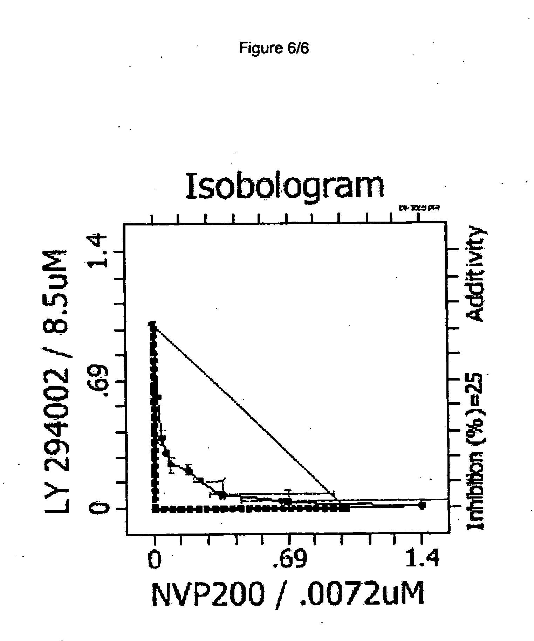

**FIG 3****:** shows the isobologram contour at 30% inhibition for the combination with 40-O-(2-hydroxyethyl)rapamycin and vinorelbine in A549 cells

### Detailed Description of the Invention

### I. The mTOR Inhibitor

### DETAILED DESCRIPTION OF THE INHIBITOR

"mTOR inhibitor" as used herein, refers to 40-O-(2-hydroxy)-ethyl-rapamycin (everolimus).
Rapamycin derivative refers to a compound of formula 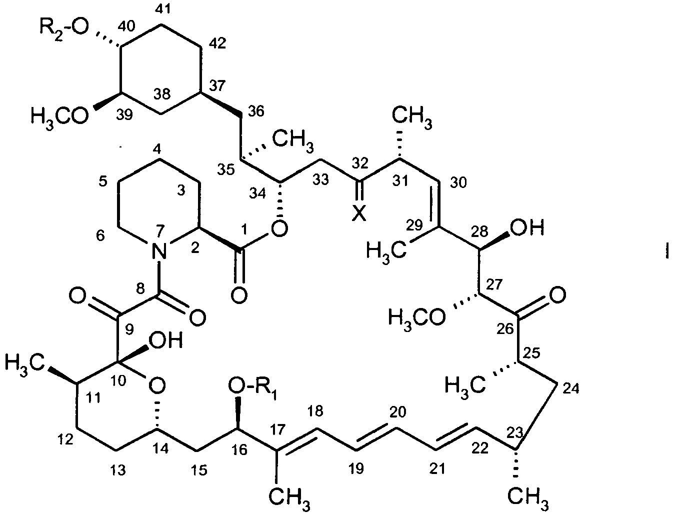 wherein
R₁ is CH₃,
R₂ is -CH₂-CH₂-OH, and
X is =O
which is 40-O-(2-hydroxy)-ethyl-rapamycin.

The compound of formula I has, on the basis of observed activity, e.g. binding to macrophilin-12 (also known as FK-506 binding protein or FKBP-12), been found to be useful as immunosuppressant, e.g. in the treatment of acute allograft rejection and as having potent antiproliferative properties which make them useful for cancer chemotherapy, particularly of solid tumors, especially of advanced solid tumors.

### II. The Pharmaceutically Active Agents

The term " pharmaceutically active agents" in the present disclosure is a broad one covering many pharmaceutically active agents having different mechanisms of action. Combinations according to the present invention can result in improvements in cancer therapy. Generally, pharmaceutically active agents are classified according to the mechanism of action. Many of the available agents are anti-metabolites of development pathways of various tumors, or react with the DNA of the tumor cells. There are also agents which inhibit enzymes, such as topoisomerase I and topoisomerase II, or which are antimiotic agents.

The term "a microtubule binding agents", as used herein, refers to a compound which acts by disrupting the microtubular network that is essential for mitotic and interphase cellular function. The microtubule agent according to the combinations of the present invention is vinorelbine.

Comprised are likewise the corresponding salts, stereoisomers, as well as corresponding crystal modifications, e.g., solvates and polymorphs, e.g. such as disclosed therein. The compounds used as active ingredients in the combination according to the present invention can be prepared and administered as described in the cited documents, respectively.

It will be understood that references to the components (a) and (b) are meant to also include the pharmaceutically acceptable salts of any of the active substances. If active substances comprised by components (a) and/or (b) have, for example, at least one basic center, they can form acid addition salts. Corresponding acid addition salts can also be formed having, if desired, an additionally present basic center. Active substances having an acid group, e.g., COOH, can form salts with bases. The active substances comprised in components (a) and/or (b) or a pharmaceutically acceptable salts thereof may also be used in form of a solvate, such as a hydrate or other solvates originating from solvents used for crystallization. 40-O-(2-hydroxyethyl)-rapamycin, is the combination partner (a).

### III. The Combinations

The present invention provides a combination comprising:
(a) everolimus; and
(b) a microtubule binding agent which is vinorelbine.

In another preferred embodiment, the present invention provides a combination comprising:
(a) a mTOR inhibitor compound of formula I; and
(b) an active agent which is Vinorelbine.

The present invention provides a combination comprising:
(a) 40-O-(2-hydroxyethyl)rapamycin; and
(b) a pharmaceutically active agent which is Vinorelbine.

The use of the combination of components (a) and (b), for the manufacturing of a medicament for treating a warm-blooded animal comprising administering these two components, a pharmaceutical composition comprising these two components for simultaneous, separate or sequential use, the use of the combination for use in the manufacture of a medicament for the delay of progression or the use in the manufacture of a medicament for the treatment of a proliferative disease or for the manufacture of a pharmaceutical preparation for these purposes or a commercial product comprising such a combination of components (a) and (b), all as mentioned or defined above, will be referred to subsequently also as COMBINATION OF THE INVENTION (so that this term refers to each of these embodiments which thus can replace this term where appropriate).

In another aspect the present invention provides the use of a combination provided by the present invention

### IV. Administration

Simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points, preferably meaning that the components (a) and (b) are administered such that no overlap of measurable blood levels of both compounds are present in an overlapping manner (at the same time).

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

The term "delay of progression" as used herein means administration of the combination to patients being in a pre-stage or in an early phase, of the first manifestation or a relapse of the disease to be treated, in which patients, e.g., a pre-form of the corresponding disease is diagnosed or which patients are in a condition, e.g., during a medical treatment or a condition resulting from an accident, under which it is likely that a corresponding disease will develop.

"Jointly therapeutically active" or "joint therapeutic effect" means that the compounds may be given separately (in a chronically staggered manner, especially a sequence-specific manner) in such time intervals that they preferably, in the warm-blooded animal, especially human, to be treated, still show a (preferably synergistic) interaction (joint therapeutic effect). Whether this is the case, can inter alia be determined by following the blood levels, showing that both compounds are present in the blood of the human to be treated at least during certain time intervals.

"Pharmaceutically effective" preferably relates to an amount that is therapeutically or in a broader sense also prophylactically effective against the progression of a proliferative disease.

### V. Commercial Package

The term "a commercial package" or "a product" or "a pharmaceutical package", as used herein defines especially a "kit of parts" in the sense that the components (a) and (b) as defined above can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (chronically staggered, in time-specific sequence, preferentially) or (less preferably) separate use thereof in the delay of progression or treatment of a proliferative disease. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) (as can be determined according to standard methods. The ratio of the total amounts of the combination partner (a) to the combination partner (b) to be administered in the combined preparation can be varied, e.g., in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g., less side effects or a combined therapeutic effect in a non-effective dosage of one or both of the combination partners (components) (a) and (b), and very preferably a strong synergism of the combination partners (a) and (b).

Both in the case of the use of the combination of components (a) and (b) and of the commercial package, any combination of simultaneous, sequential and separate use is also possible, meaning that the components (a) and (b) may be administered at one time point simultaneously, followed by administration of only one component with lower host toxicity either chronically, e.g., more than 3-4 weeks of daily dosing, at a later time point and subsequently the other component or the combination of both components at a still later time point (in subsequent drug combination treatment courses for an optimal anti-tumor effect) or the like.

The COMBINATION OF THE INVENTION can also be applied in combination with other treatments, e.g. such as usual in preventing or treating proliferative diseases, such as surgical intervention, hyperthermia and/or irradiation therapy.

### VI. Pharmaceutical Compositions & Preparations

The pharmaceutical compositions according to the present invention can be prepared by conventional means and are those suitable for enteral, such as oral or rectal, and parenteral administration to mammals including man, comprising a therapeutically effective amount of a VEGF inhibitor and at least one pharmaceutically active agent alone or in combination with one or more pharmaceutically acceptable carriers, especially those suitable for enteral or parenteral application.

The pharmaceutical compositions comprise from about 0.00002 to about 100%, especially, e.g., in the case of infusion dilutions that are ready for use) of 0.0001 to 0.02%, or, e.g., in case of injection or infusion concentrates or especially parenteral formulations, from about 0.1 % to about 95%, preferably from about 1% to about 90%, more preferably from about 20% to about 60% active ingredient (weight by weight, in each case). Pharmaceutical compositions according to the invention may be, e.g., in unit dose form, such as in the form of ampoules, vials, dragées, tablets, infusion bags or capsules.

The effective dosage of each of the combination partners employed in a formulation of the present invention may vary depending on the particular compound or pharmaceutical compositions employed, the mode of administration, the condition being treated and the severity of the condition being treated. A physician, clinician or veterinarian of ordinary skill can readily determine the effective amount of each of the active ingredients necessary to prevent, treat or inhibit the progress of the condition.

However, in general, for satisfactory results in larger mammals, for example humans, an indicated daily dosage includes a range
- from about 0.0001 g to about 1.5 g, such as 0.001 g to 1.5 g;
- from about 0.001 mg/kg body weight to about 20 mg/kg body weight, such as 0.01 mg/kg body weight to 20 mg/kg body weight,
for example administered in divided doses up to four times a day.

In a combination of the present invention, rapamycin or a rapaymcin derivative may be administered as appropriate, e.g. in dosages which are known for compounds of the present invention, by any administration route, e.g. enterally, e.g. orally, or parenterally. E.g. everolimus may be administered, e.g. orally, in dosages from 0.1 mg up to 15 mg, such as 0.1 mg to 10 mg. e.g. 0.1 mg, 0.25 mg, 0.5 mg, 0.75 mg, 1 mg, 2.5 mg, 5 mg, or 10 mg, more preferably from 0.5 mg to 10 mg, e.g. in the form of (dispersible) tablets; e.g. comprising everolimus in the form of a solid dispersion; e.g. a weekly dosage may include up to 70 mg, e.g. 10 to 70 mg, such as 30 to 50 mg, e.g. depending on the disease being treated.

Pharmaceutical preparations for the combination therapy for enteral or parenteral administration are, e.g., those in unit dosage forms, such as sugar-coated tablets, capsules or suppositories, and furthermore ampoules. If not indicated otherwise, these formulations are prepared by conventional means, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units. One of skill in the art has the ability to determine appropriate pharmaceutically effective amounts of the combination components.

Preferably, the compounds or the pharmaceutically acceptable salts thereof, are administered as an oral pharmaceutical formulation in the form of a tablet, capsule or syrup; or as parenteral injections if appropriate.

In preparing compositions for oral administration, any pharmaceutically acceptable media may be employed such as water, glycols, oils, alcohols, flavoring agents, preservatives, coloring agents. Pharmaceutically acceptable carriers include starches, sugars, microcrystalline celluloses, diluents, granulating agents, lubricants, binders, disintegrating agents.

Solutions of the active ingredient, and also suspensions, and especially isotonic aqueous solutions or suspensions, are useful for parenteral administration of the active ingredient, it being possible, e.g., in the case of lyophilized compositions that comprise the active ingredient alone or together with a pharmaceutically acceptable carrier, e.g., mannitol, for such solutions or suspensions to be produced prior to use. The pharmaceutical compositions may be sterilized and/or may comprise excipients, e.g., preservatives, stabilizers, wetting and/or emulsifying agents, solubilizers, salts for regulating the osmotic pressure and/or buffers, and are prepared in a manner known per se, e.g., by means of conventional dissolving or lyophilizing processes. The solutions or suspensions may comprise viscosity-increasing substances, such as sodium carboxymethylcellulose, carboxymethylcellulose, dextran, polyvinylpyrrolidone or gelatin. Suspensions in oil comprise as the oil component the vegetable, synthetic or semi-synthetic oils customary for injection purposes.

The isotonic agent may be selected from any of those known in the art, e.g. mannitol, dextrose, glucose and sodium chloride. The infusion formulation may be diluted with the aqueous medium. The amount of aqueous medium employed as a diluent is chosen according to the desired concentration of active ingredient in the infusion solution. Infusion solutions may contain other excipients commonly employed in formulations to be administered intravenously such as antioxidants.

The present invention further relates to "a combined preparation", which, as used herein, defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g., in order to cope with the needs of a patient sub-population to be treated or the needs of the single patient based on the severity of any side effects that the patient experiences.

The present invention especially relates to a combined preparation which comprises:
(a) one or more unit dosage forms of the mTOR inhibitor everolimus; and
(b) one or more unit dosage forms of an pharmaceutically active agent which is vinorelbine.

### VII. The Diseases to be Treated

The compositions of the present invention are useful for the manufacture of a medicament for treating proliferative diseases or diseases that are associated with or triggered by persistent angiogenesis.

A proliferative disease is mainly a tumor disease (or cancer) (and/or any metastases). The inventive compositions are particularly useful for the manufacture of a medicament for treating a tumor which is a breast cancer, genitourinary cancer, lung cancer, gastrointestinal cancer, epidermoid cancer, melanoma, glioma, ovarian cancer, pancreas cancer, neuroblastoma, head and/or neck cancer or bladder cancer, or in a broader sense renal, brain or gastric cancer.

In particular, the inventive compositions are particularly useful for the manufacture of a medicament for treating:
(i) a breast tumor; a lung tumor, e.g., a small cell or non-small cell lung tumor; melanoma; or
(ii) a proliferative disease that is refractory to the treatment with other chemotherapeutics; or
(iii) a tumor that is refractory to treatment with other chemotherapeutics due to multidrug resistance.

Where a tumor, a tumor disease, a carcinoma or a cancer are mentioned, also metastasis in the original organ or tissue and/or in any other location are implied alternatively or in addition, whatever the location of the tumor and/or metastasis.

The composition are selectively toxic or more toxic to rapidly proliferating cells than to normal cells, particularly in human cancer cells, e.g., cancerous tumors, the compound has significant anti-proliferative effects and promotes differentiation, e.g., cell cycle arrest and apoptosis.

The invention is illustrated by the following Examples.

The following Examples illustrate the combinations with 40-O-(2-hydroxyethyl)-rapamycin that show a syngeristic effect. All combinations were tested in three (3) distinct cell lines as part of this collaboration: A549, a model of non-small cell lung carcinoma; SKOV-3, a model of ovarian cancer; and SKMEL-28, a model of malignant melanoma.

One example is the synergistic effect observed between 40-O-(2-hydroxyethyl)-rapamycin and vinorelbine in A549 cells. In this combination, an increase in the maximum effect was observed compared to either of the single agents alone.

All combinations are prepared in the same manner for testing.

### Assay conditions and protocols

### Day 1: Cell preparation

Cells were cultured in T-175 flasks in complete medium (RPMI-1640, 10% FBS, 1% Penn/Strep) at 37 °C and 5% CO2. Cells were removed from the flask by brief treatment with 0.25% trypsin. Trypsin was inactivated with media and cell count was adjusted appropriately. Cells were then seeded into 384-well microtiter plates (35µL) at 1500 (A549) or 3,000 (SKOV-3, SKMEL-28) cells/well using a multi-drop 16-24 hours prior to compound addition for general screening. Seeded plates were incubated (37 °C / 5% CO2) overnight to allow recovery and re-attachment.

### Day 2: Compound addition

Dilution plates were prepared with 100 µL per well of complete medium non-cell culture treated polypropylene 384-well plates. Compounds were added to dilution plates using the Mini-Trak (1 µL addition) for a 1:101 dilution followed by mixing. For single agent dose response curves, a 5µL aliquot from a dilution plate was added to assay plates to generate the 11-point dose responsecurve (final volume 40 µL). Final dilution was ~1:808 with total solvent concentration ~0.1%. For combination matrices, 4.5 µL aliquots from dilution plates of orthogonally-titrated master plates were added to the same assay plate to generate the dose-response matrix (final volume of 44 µL). Final dilution of each compound was ~1:988 with total solvent concentration ~0.2%. After compound addition, plates were incubated at 37 °C / 5% CO2 for 72 hours.

### Day 5: Measure cell viability

A solution of 5% CellTiter-Blue (Promega) viability dye in complete medium was dispensed to assay plates using a multi-drop or 384-well pipettor. An appropriate volume was added for a final dye concentration of 2.5%. Viability reactions were incubated for 4 to 6 hours depending on cell type at 37 °C / 5% CO2 to allow reduction of viability dye. Plates were allowed to cool to room temperature for one hour before reading fluorescence intensity at 590 nm after excitation at 540 nm in a Wallac Victor-V plate reader.

| **Table III: Cell Lines, Media and Reagents** | | | |
|---|---|---|---|
| | **Source** | **Catalog** # | **Lot**# |
| **Cell Lines** | | | |
| A549 | ATCC | CCL-185 | 3449902 |
| SKMEL-28 | ATCC | HTB-72 | 348832 |
| SKOV-3 | ATCC | HTB-77 | 3898710 |

| **Medium and Reagents** | | | |
|---|---|---|---|
| Base Medium: RPMI-1640^{‡} | ATCC | 30-2001 | |
| Penicillin/Streptomycin | Cellgro | 30-002-CI | 30002098 |
| Fetal bovine serum | Gibco | 16000-044 | 1127751 |
| Trypsin-EDTA (0.25%) | Cellgro | 25-053-CI | 25053103 |
| L-glutamine | Gibco | 25030-081 | 11150 |
| Celltiter-Blue Viability Dye | Promega | G8081 | 200719 |

| | | | |
|---|---|---|---|
| ^{‡}Base medium is supplement to create complete medium: 10% FBS, Penicillin/Streptomycin (1: 100), there is no need to add L-glutamine if ATCC medium is used within 3 months after receipt. | | | |

### QC Criteria

### Primary plate QC status

cHTS plate formats contain groups of positive and negative intra-plate control wells that are used for automated quality control. All assay plates are assigned an automated QC value by the LIM system following data collection. Automatic quality control calls are made based on the Z-factor calculated using intra-plate controls using a standard factor Z = 1-3(_V+_U)/(*V*-*U*), where *V*,*U* are the mean vehicle (treated) and media (untreated) control levels, and _V,_U are the corresponding standard deviation estimates. Z-factor thresholds are empirically set to group plates into three classes: automatically accepted (Z >0.6), automatically rejected (Z <0.4), and undetermined plates that need to be visually evaluated (0.4< Z <0.6). Where necessary the QC status of accepted plates may be reassigned to rejected status based on visual inspection of plate quality, transfer controls or other secondary QC criteria. Plates rejected automatically or by visual inspection are excluded from further analysis and scheduled to be repeated.

### Transfer controls

A positive control compound (Gentian Violet) is included on all master plates. This provides a visual check for screening scientists to verify compound transfer from both column and row masters into the assay plate.

### Secondary QC

Secondary QC includes additional manual checks of data quality including: visual inspection of plate quality and transfer controls, marking of data spikes, and checking for cell-line appropriate behavior of single agents. Plates with an accepted status from primary QC that show an unacceptable plate gradient are adjusted to rejected status and queued for repeat. Plates are also visually inspected for occasional bad wells, or "spikes" with data values that are very different from their immediate neighbors (within the same treatment class). These data spikes are flagged in the database, and excluded from subsequent analyses. Finally, dose-response matrices containing single-agent activity inconsistent with past experience will be marked with rejected status and queued for repeat. Data blocks that did not achieve the cut-off threshold were flagged in the database, excluded from subsequent analysis and queued for repeat as necessary.

### Measuring Antiproliferative Activity

The measure of effect was the inhibition of cell viability using an alamar blue viability assay relative to the untreated level (vehicle alone). For untreated and treated levels U and T, a fractional inhibition I = 1-T/U was calculated. The inhibition ranges from 0% at the untreated level to 100% when T = 0.

Each treated level T was compared to the median untreated level U ± σU, determined for each plate by finding the median alamar blue level (and its associated uncertainty, described above) among the untreated control wells arranged across the plate. Applying standard error propagation rules to the expression for I, the estimated standard error σl ~ (σU/U) sqrt(1-I).

The error estimates were further increased to account for variations between replicate combination blocks as well as a minimum assumed fractional uncertainty of_min ~ 3%. Thus for inhibition, the standard error estimate becomes σl ~ sqrt{ (σU/U)2 (1-I) + σrep 2 + σₘᵢₙ².

### Medians and Error Estimates

Medians were used rather than averages to reduce the effect of occasional outliers on the consensus. While medians are more robust to outliers, they are more sensitive to statistical noise,yielding ∼30% larger deviations. Standard deviations are estimated from the median absolute deviation (MAD), where for a normal distribution, the sample deviation σdat ∼ 1.5 MAD. The standard error for the median itself is then σmed ∼ σdat/sqrt(N-1), given N data values.

### Single Agent Dose Curves

The single agent activity is characterized by fitting a sigmoidal function of the form I = Imax/[1+(C/EC50) °], with least squares minimization using a downhill simplex algorithm.Here, C is the concentration, EC50 is the effective concentration at 50% inhibition, and σ is the sigmoidicity. The uncertainty of each fitted parameter was estimated from the range over which the change in reduced chi-squared X² is less than one, or less than minimum reduced X 2 if that minimum exceeds one, to allow for underestimated σl errors. To ensure optimal concentration the EC50 was determined and maximum effect level in each of the proposed proliferation assays. 384-well plates were used , to obtain duplicate dose response curves in 12-step dilutions with a dosing ratio f = 2, 3, or 4, to cover 3-7 orders of magnitude.

### Selecting Optimal Concentrations

We use the single agent curve data to define a dilution series for each compound to be used for combination screening. Using a dilution factor f of 2, 3, or 4, depending on the sigmoidicity of the single agent curve, we will choose 5 dose levels with the central concentration close to the fitted EC50. For compounds with no detectable single agent activity, we will use f = 4 starting from the highest achievable concentration.

### Combination Dose Matrices and Reference Models

The cHTS screening produces dose matrices which contain aii pairwise combinations of two single agents at a series of concentrations, including zero. Each dose matrix contains internal copies of the single agent curves which are used as the reference for combination effects. Replicate dose matrices can be merged together by medianing the corresponding data points, and when the concentration series differ, corresponding values are found using bilinear interpolation. Standard errors were computed for each inhibition value using the formulas described above. Combination effects were most readily characterized by comparing each data point's inhibition to that of a combination reference model that was derived from the single agent curves. Three models are generally used: (1) The highest single agent model IHSA(CX,CY) = max(IX,IY) is a simple reference model, where CX,Y are the concentrations of the X and Y compound, and IX,Y are the inhibitions of the single agents at CX,Y; (2) Bliss independence IBliss(CX,CY) = IX + IY - IXIY represents the statistical expectation for independent competing inhibitors; and (3) Loewe additivity, where ILoewe(CX,CY) is the inhibition that satisfies (CX/ECX) + (CY/ECY) = 1, and ECX,Y are the effective concentrations at ILoewe for the single agent curves. Loewe additivity is the generally accepted reference for synergy[4], as it represents the combination response generated if X and Y are the same compound. Both IHSA and IBliss are easily calculated from IX,Y, but determining ILoewe requires interpolation and numerical root finding.

### Selecting Combinations for 9x9 Re-test

To select desirable oncology combinations for repeat assays using high resolution 9x9 dose matrices, three important considerations were evaluated: (1) significant synergy over the additive model; (2) substantial activity where the synergy occurs; and (3) sufficient potency shifting. A "Synergy Score"was used whereby S = log fX log fY_Idata (Idata-ILoewe), summed over all non-single-agent concentration pairs, and where log fX,Y are the natural logarithm of the dilution factors used for each single agent. This effectively calculates a volume between the measured and Loewe additive response surfaces, weighted towards high inhibition and corrected for varying dilution factors. This volume score emphasizes the overall synergistic or antagonistic effect of the combination, thus minimizing the effects of outlying data spikes and identifying combinations with a robust synergy across a wide range of concentrations and at high effect levels. S is positive for mostly synergistic combinations and negative for antagonism. In cases where both syn rgy and antagonism are present at different concentrations, the weighting favors effects at high inhibition levels. An uncertainty σS is calculated for each synergy score, based on the measured errors for the Idata values and standard error propagation. The synergy score was used and its error to define an appropriate selection cutoff. For example, combinations with S > 2_S are significant at ~95% confidence, assuming a normal distribution. Also, to ensure a sufficient potency shift, the combination index, Cl = (CX/ECX) + (CY/ECY) at a chosen effect level is small enough to represent a useful synergy. Observed *in vitro* Cl measurements for currently used clinical combinations (Cl~ 0.5-0.7) can be used as a guide in setting the cutoff.

The Table below lists the combinations of vinorelbine with 40-O-(2-hydroxyethyl)-rapamycin

| Combination | Synergy Score | Cell Line |
|---|---|---|
| | | |
| | | |
| | | |
| | | |
| 40-O-(2-hydroxyethyl)-rapamycin + Carboplatin + vinorelbine | 1.475 | A549 |
| | | |
| | | |
| | | |
| | | |
| | | |
| | | |
| | | |
| | | |
| Vinorelbine | 0.887 | SKOV3 A549 |
| | | |
| | | |

## Claims

1. A combination of
(a) everolimus; and
(b) vinorelbine.

2. A combination of claim 1 for simultaneous, concurrent, separate or sequential use in preventing or treating a proliferative disease.

3. A pharmaceutical composition comprising a combination according to claim 1.

4. A commercial package comprising a combination according to claim 1.

5. A commercial package of claim 4, wherein the unit dosage form is a fixed combination.

6. The use of a combination according to claim 1 or 2, pharmaceutical composition according to claim 3 or commercial package according claim 4 or 5, for the manufacture of a medication for preventing or treating proliferative diseases, wherein the proliferative disease is breast cancer, ovarian cancer, lung carcinoma, non-small cell lung cancer or melanoma.

## Patentansprüche

1. Kombination von
(a) Everolimus; und
(b) Vinorelbin.

2. Kombination nach Anspruch 1 zur simultanen, gleichzeitigen, getrennten oder aufeinanderfolgenden Verwendung bei der Prävention oder Behandlung einer proliferativen Erkrankung.

3. Pharmazeutische Zusammensetzung, umfassend eine Kombination nach Anspruch 1.

4. Handelsübliche Packung, umfassend eine Kombination nach Anspruch 1.

5. Handelsübliche Packung nach Anspruch 4, wobei die Einheitsdosierungsform eine feste Kombination ist.

6. Verwendung einer Kombination nach Anspruch 1 oder 2, einer pharmazeutischen Zusammensetzung nach Anspruch 3 oder einer handelsüblichen Packung nach Anspruch 4 oder 5 zur Herstellung eines Medikaments zur Prävention oder Behandlung von proliferativen Erkrankungen, wobei die proliferative Erkrankung Brustkrebs, Eierstockkrebs, Lungenkarzinom, nicht-kleinzelliger Lungenkrebs oder Melanom ist.

## Revendications

1. Combinaison
(a) d' éverolimus ; et
(b) de vinorelbine.

2. Combinaison selon la revendication 1, pour emploi simultané, concomitant, séparé ou séquentiel pour prévenir ou pour traiter une maladie proliférative.

3. Composition pharmaceutique comprenant une combinaison selon la revendication 1.

4. Produit commercial comprenant une combinaison selon la revendication 1.

5. Produit commercial selon la revendication 4, dans lequel la forme de dosage unitaire est une combinaison fixe.

6. Emploi d'une combinaison selon la revendication 1 ou 2, composition pharmaceutique selon la revendication 3 ou produit commercial selon la revendication 4 ou 5, pour la fabrication d'un médicament pour prévenir ou pour traiter des maladies prolifératives, la maladie proliférative étant un cancer du sein, un cancer de l'ovaire, un carcinome du poumon, un cancer du poumon non à petites cellules ou un mélanome.
